# FASCICULE DE BREVET EUROPEEN

(11) **EP 2 675 375 B1**
(45) Date de publication et mention de la délivrance du brevet: **08.06.2016**
(21) Numéro de dépôt: 12708913.4
(22) Date de dépôt: 13.02.2012
(51) Int. Cl.: A61B 17/70, A61B 17/88

(54) **DISPOSITIF CHIRURGICAL POUR LA CORRECTION DE LA DEFORMATION DE LA COLONNE VERTEBRALE**
CHIRURGISCHE VORRICHTUNG ZUR KORREKTUR VON VERFORMUNGEN DER WIRBELSÄULE
SURGICAL DEVICE FOR CORRECTING DEFORMATION OF THE SPINAL COLUMN

(30) Priorité: 18.02.2011 FR 1151331
(43) Date de publication de la demande: 25.12.2013
(73) Titulaire: Spineway, 69130 Ecully (FR)
(72) Inventeur: MARUENDA PAULINO, José Ignacio, E-46010 Valence (ES); LAURITO, Philippe, F-83143 Le Val (FR); LE ROUX, Stéphane, F-69002 Lyon (FR)
(74) Mandataire: Cabinet Laurent & Charras
(86) Numéro de dépôt international: PCT/FR2012/050310
(87) Numéro de publication internationale: WO 2012/123655

(56) Documents cités:
- WO-A1-2010/047736
- WO-A2-2006/104813
- US-A1- 2009 005 814

## Description

L'invention se rattache au secteur technique des instruments chirurgicaux pour la correction d'une déformation de la colonne vertébrale.

L'invention trouve une application particulièrement avantageuse pour la correction d'une scoliose.

Dans le domaine de l'ostéosynthèse rachidienne, il est parfaitement connu d'utiliser des vis pédiculaires dont les têtes, sous forme de douilles, présentent des agencements pour le montage et la fixation d'au moins une tige de liaison pour réaliser une distraction ou compression, et redéfinir la courbure.

Différentes solutions techniques ont été proposées, comme il ressort, par exemple, de l'enseignement des brevets FR 2.658.413 et EP 0.699.056.

Quelle que soit la solution technique choisie pour la mise en place des tiges de liaison, il est nécessaire que les têtes de vis pédiculaires soient sensiblement alignées pour l'engagement desdites tiges au travers d'échancrures que présentent les têtes de vis.

Or, dans les pathologies de la colonne à type de déformation sévère (comme les scolioses) les vertèbres se trouvent déplacées dans les trois plans de l'espace : rotation, décalage et bascule

Il s'avère donc nécessaire de pouvoir corriger cette déformation de la colonne vertébrale avec, pour objectif, de redresser les vertèbres en vue de leur alignement.

Là encore, différentes solutions techniques ont été proposées.

Le concept de base de ces différentes solutions est l'utilisation de tubes que l'on fixe temporairement sur les têtes de vis pédiculaires. Les différents tubes sont donc fixés perpendiculairement à l'axe de la colonne et sont décalés angulairement, compte tenu de la déformation de la colonne. Il convient donc de redresser ces tubes en vue de leur alignement, pour corriger la rotation des vertèbres, afin de remédier à la correction de la déformation de la colonne vertébrale.

L'invention concerne plus particulièrement le principe de base de correction tel que défini, par exemple, dans la revue « SPINE » Volume 33, 2008 N° 14, pages 1588 à 1597, principe qui, par ailleurs, a fait l'objet d'une protection par brevet, comme il ressort de l'enseignement du document WO 2006/104813 qui divulgue le préambule de la revendication 1.

Pour l'essentiel, des tiges sont engagées au travers des différents tubes en étant progressivement déplacées de haut en bas pour sensiblement aligner lesdits tubes correspondant à la correction de la colonne vertébrale.

Selon l'état de la technique, les tubes sont fixés à l'intérieur des têtes une rotation des vertèbres, mais ne permettent pas d'implanter la tige définitive de correction destinée à relier, comme indiqué, les têtes de vis pédiculaires.

En effet, selon l'état de la technique, après avoir descendu les tiges introduites au travers de plusieurs tubes, sensiblement perpendiculairement à leur génératrice, afin de réaliser l'alignement, l'opérateur est obligé d'aller de l'autre côté de la colonne, par exemple du côté concave, si ce dernier a commencé l'opération du côté convexe. Une fois que la tige implantable a été fixée du côté concave, l'opérateur est obligé de démonter les éléments tubulaires pour fixer la tige implantable du côté convexe.

Il est donc nécessaire de procéder en plusieurs fois, ce qui présente certains inconvénients. En effet, outre la perte de temps en résultant, la nécessité d'assurer le montage de la tige implantable du côté opposé à celui recevant l'instrumentation pour la correction de la colonne, fait perdre une quantité de la correction.

L'invention s'est fixée pour but de remédier à ces inconvénients d'une manière simple, sûre, efficace et rationnelle.

Le problème que se propose de résoudre l'invention est de pouvoir, en une seule fois, intervenir d'un côté de la colonne, côté convexe par exemple, puis de l'autre côté de cette colonne, côté concave, en assurant la double fonction, d'une part, de redresser les corps vertébraux au moyen d'éléments tubulaires et, d'autre part, de positionner la tige implantable et la fixer au niveau des têtes de vis pédiculaires, sans être obligé de démonter lesdits éléments tubulaires, tout en ayant pour objectif de déterminer la courbure de type cyphose thoracique à restaurer, dans un plan sagittal.

Pour résoudre un tel problème, il a été conçu et mis au point un dispositif chirurgical conforme aux caractéristiques de la revendication 1.

Ces caractéristiques permettent donc, d'une part, de corriger la rotation des vertèbres et de maintenir en place la correction obtenue, et, d'autre part, de mettre en place la tige implantable, puis le verrouillage de ladite tige sur les vis par des écrous de fixation introduits dans les éléments tubulaires de correction sans perdre la position relative desdits éléments, et ce sans aucun démontage.

Pour résoudre le problème posé de pouvoir accoupler les éléments tubulaires à l'extérieur des têtes de vis avec, pour objectif, de totalement libérer l'intérieur desdites têtes, chaque élément tubulaire est en deux parties indépendantes, l'une des extrémités de chaque partie présentant des agencements d'accouplement desdites parties entre elles et par rapport à la tête de vis pédiculaire correspondante.

Les agencements d'accouplement peuvent être constitués par deux fourchettes aptes à être reliées avec capacité d'articulation pour permettre d'écarter les parties pour leur mise en place par rapport à la tête de vis pédiculaire puis les rabattre, afin d'enserrer ladite tête maintenue entre lesdites parties par des moyens de retenue.

Pour résoudre le problème de constituer l'élément tubulaire en tant que tel, pour l'engagement des différentes tiges, l'autre extrémité de chaque partie peut coopérer, après avoir été rabattues, avec un bouchon creux de liaison pour constituer l'élément tubulaire en tant que tel, lesdites parties délimitant les lumières diamétralement opposées.

Pour résoudre le problème d'assurer la mise en place de la tige cintrée implantable au moyen de la tige de déplacement (appelée tige) de manoeuvre)engagée au travers de plusieurs tubes avec capacité de déplacement vertical de haut en bas, le moyen de déplacement de ladite tige cintrée implantable peut être une bague montée avec capacité de coulissement le long de l'élément tubulaire considéré.

Pour résoudre le problème d'indexer angulairement la tige cintrée au fur et à mesure de son déplacement en direction des têtes de vis pédiculaires, la bague peut présenter, à sa base, des échancrures diamétralement opposées aptes à être positionnées en regard des lumières pour coopérer avec la section de la tige cintrée.

Pour résoudre le problème de la mise en place des tiges d'alignement et de manoeuvre, et leur maintien en position d'attente, les échancrures de la bague peuvent coopérer avant déplacement de ladite bague, avec un ergot de blocage en position, les tiges d'alignement et de manoeuvre)étant disposées au-dessus de la bague, tandis que la tige cintrée implantable est disposée sous ladite bague.

Pour résoudre le problème posé de pouvoir accoupler, à l'extérieur des éléments tubulaires, la tige de manoeuvre, afin de dégager le passage de l'écrou de serrage, l'extrémité de chaque élément tubulaire, à l'opposé de son accouplement avec la tête de vis pédiculaire, reçoit un collier mobile rapporté qui peut présenter une partie débordant latéralement pour l'engagement de la tige de manoeuvre ayant servi à déplacer la bague et, d'une manière concomitante, la tige cintrée implantable.

En ayant pour objectif de déterminer la position angulaire des vertèbres dans un plan sagittal, notamment afin de restaurer une courbure physiologique (cyphose thoracique, lordose lombaire) et de parfaitement maintenir en position alignée les différents éléments tubulaires, ces derniers peuvent être reliés deux à deux au niveau de leur extrémité libre, par des organes espaceurs.

Pour résoudre le problème de pouvoir introduire un écrou de fixation dans la tête de vis pédiculaire, en vue de la fixation de la tige cintrée implantable, la tête de vis pédiculaire qui présente, d'une manière connue, une échancrure en constituant une douille pour l'engagement de la tige cintrée, peut être prolongée, de part et d'autre de l'échancrure, par des pattes sécables et taraudées pour la mise en place de l'écrou en vue de la fixation de la tige cintrée de la tête de vis pédiculaire.

Selon une autre caractéristique, la tête de vis pédiculaire peut être du type monobloc ou polyaxial.

En considérant les caractéristiques du dispositif revendiqué la correction d'une déformation de la colonne vertébrale s'effectue comme suit :
- on accouple préalablement des vis pédiculaires et des éléments tubulaires, réalisés en deux parties pour enserrer la face externe des têtes de vis ;
- on implante les vis pédiculaires équipées des éléments tubulaires qui font office de tournevis démontable et réutilisable ;
- on engage transversalement une tige d'alignement au travers des différents éléments tubulaires alignés manuellement sans difficulté pour provoquer l'alignement desdits éléments, correspondant à la correction de la colonne, suite à une rotation des vertèbres ;
- on accouple les différents éléments tubulaires par un organe espaceur ;
- on engage transversalement, sous la tige d'alignement, une tige de manoeuvre apte à prendre un appui sur un moyen monté avec capacité de déplacement guidé le long de l'élément tubulaire;
- on engage transversalement, sous le moyen déplaçable le long du tube, une tige cintrée implantable destinée à être fixée dans les têtes de vis pédiculaires ;
- on agit en poussée, selon un mouvement dirigé de haut en bas sur la tige de manoeuvre, pour provoquer le déplacement du moyen pour permettre le déplacement concomitant de la tige cintrée et son positionnement dans les têtes de vis ;
- on retire la tige de manoeuvre que l'on positionne dans des agencements externes que présentent chacun des éléments tubulaires ;
- on retire la tige d'alignement ;
- on engage un écrou et un organe de manoeuvre à l'intérieur de chaque élément tubulaire pour assurer la fixation de la tige cintrée dans chacune des têtes de vis pédiculaires correspondantes ;
- on retire les éléments tubulaires.

La colonne étant alignée par cette implantation d'un coté (Montage unilatérale), on peut procéder à la mise en place d'implants du coté laissé libre de la colonne vertébrale (Montage bilatéral).
On peut également prévoir la mise en place, soit simultanée soit l'un après l'autre, de vis-tubes de l'autre coté des vertèbres, on pourra ainsi réalisé un montage symétrique bilatéral. En augmentant le nombre de vis équipées de tubes pour un nombre identique de vertèbres, on obtient donc plus de contrôle, moins de pression unitaire sur chaque vis/pédicule, compte tenu d'une répartition des forces entre un plus grand nombre de tubes-vis.

Parmi d'autres avantages de l'invention, on peut citer :

Après avoir procédé aux étapes précédemment décrites, et mis en place une tige cintrée dans les têtes de vis pédiculaires maintenues par des écrous de serrage non vissés à fond (non bloqués sur ladite tige) :
- On enlève la tige de manoeuvre (dans les oeillets à l'extérieur du montage).
- On enlève la tige d'alignement.
- On insère, dans la lumière des éléments tubulaires, une nouvelle tige de manoeuvre plus courte.

L'emploi d'une tige de manoeuvre plus courte permet de solidariser un plus petit nombre d'éléments tubulaire et de vertèbres, au choix de l'opérateur pendant l'intervention. Il est par conséquent possible de manoeuvrer certaines vertèbres seulement, tout en en maintenant d'autres en position fixe.

Par exemple un ou deux éléments tubulaires et vertèbres maintenus fixes à chaque extrémité d'un montage, permettent de réaliser un point fixe par rapport auquel l'opérateur pourra manoeuvrer les éléments tubulaires du centre solidarisés entre eux par une tige dont la longueur correspond exactement à l'écartement des éléments tubulaires que l'ont veut manipuler.

Ceci correspond à un objectif de l'intervention chirurgicale qui est de corriger la gibbosité, à savoir un groupe de cotes, déformé plus que ne voudrait le déplacement des vertèbres qui y sont attachées, et qui forment une bosse dans le dos.

On peut solidariser ou désolidariser, de façon adaptée aux besoins de correction de gibbosité observés pendant l'intervention, des groupes de tubes-vis-vertèbres, en faisant varier la longueur de la tige de manoeuvre.

L'invention est exposée ci-après plus en détail à l'aide des figures des dessins annexés dans lesquels :
- la figure 1 est une vue en perspective d'un exemple de vis pédiculaire utilisée avec le dispositif chirurgical de correction selon l'invention ;
- les figures 2 et 3 sont des vues à caractère purement schématique montrant une déformation de colonne sous forme d'une scoliose avant correction (figure 2) et après correction (figure 3) ;
- les figures 4 à 20 sont des vues en perspective montrant les principales étapes de la correction d'une colonne vertébrale au moyen du dispositif chirurgical selon l'invention, les corps vertébraux de ladite colonne étant symbolisés par des cylindres ;

- la figure 21 est une vue à caractère schématique montrant la mise en place du dispositif des deux côtés de la colonne ;
- la figure 22 est une vue en perspective montrant la mise en place de l'élément tubulaire au niveau de la tête de vis pédiculaire.

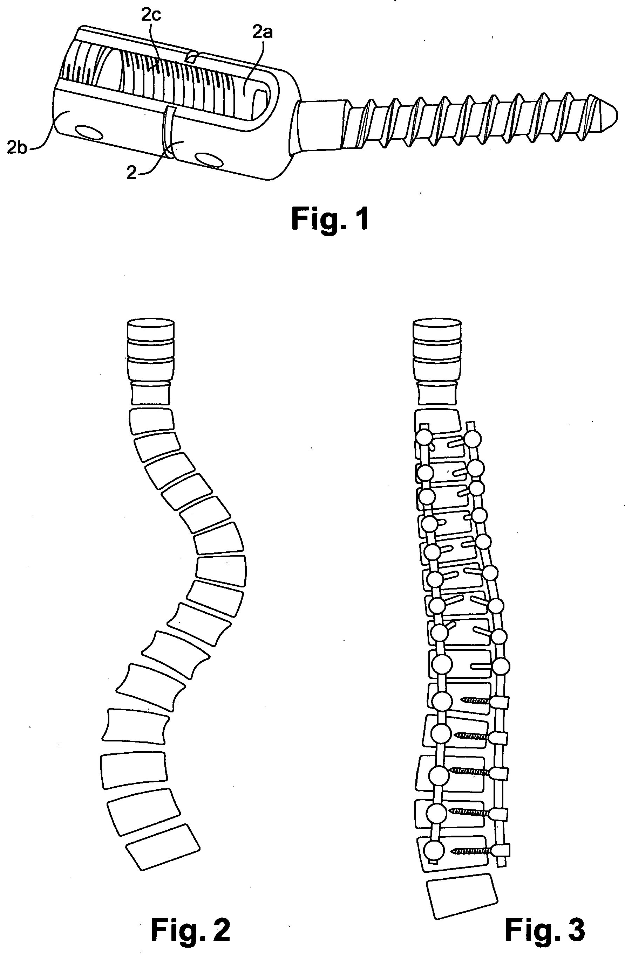

On a illustré aux figures 2 et 3 le principe de correction de la colonne vertébrale. La déformation présentée correspond, par exemple, à une scoliose. Comme indiqué, les différentes vertèbres (V) sont symbolisées par des cylindres.

On rappelle, d'une manière connue, que le dispositif chirurgical pour effectuer cette correction comprend des éléments tubulaires (1) aptes à être fixés temporairement au niveau des têtes de vis pédiculaires (2) agencées pour être reliées par une tige cintrée implantable (3) de distraction ou de compression. Des tiges (4) et (5) sont destinées à être engagées au travers des différents éléments tubulaires (1). La tige (4) constitue une tige d'alignement pour aligner les éléments tubulaires (1), en vue d'assurer la correction de la colonne vertébrale suite à une rotation, un décalage et une bascule, ceci dans les trois plans de l'espace, des vertèbres résultant dudit alignement. La tige (5) constitue une tige de manoeuvre pour la mise en place de la tige implantable (3) dans la tête de vis pédiculaire (2).

On rappelle que le but recherché selon l'invention, est de pouvoir aligner les différents éléments tubulaires (1) conduisant au redressement des différentes vertèbres, puis, dans le même temps opératoire, sans aucun démontage, mettre en place la tige cintrée de correction implantable (3) dans les différentes têtes de vis (2) et fixer ladite tige cintrée (3) dans lesdites têtes de vis (2). A noter qu'il n'y a pas de perte de la correction obtenue, l'insertion de la tige cintrée se produit au centre des éléments tubulaires de guidage.

Selon une première caractéristique, chaque élément tubulaire (1) présente des agencements pour être accouplé à l'extérieur des têtes de vis pédiculaires (2)

A noter que la forme de la partie inférieure de chaque élément tubulaire possède une très large surface de contact avec les faces extérieures de la tête de vis, ce qui permet une importante solidarité et cohésion entre les éléments tubulaires et les vis, afin de déplacer, corriger et contrôler le déplacement de la position des vertèbres pour rectifier la déformation

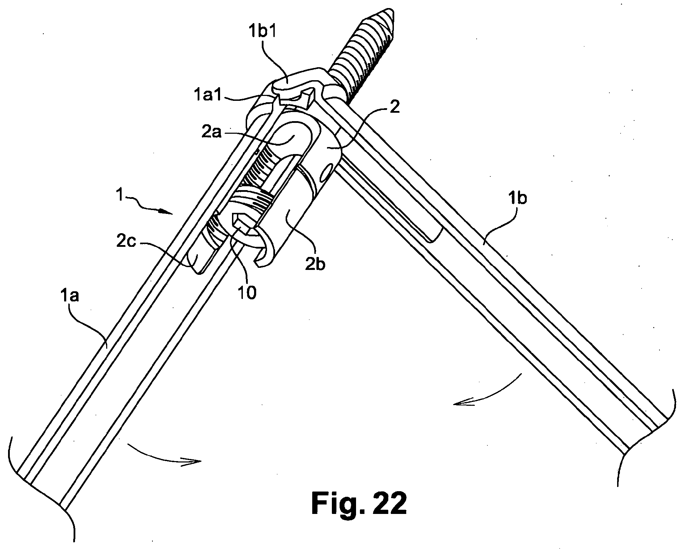

Dans ce but, et comme le montre la figure 22, chaque élément tubulaire (1) est en deux parties indépendantes (1a) et (1b). L'une des extrémités de chaque partie (1a) et (1b) présente des agencements (1a1) est (1b1) conformés pour assurer l'accouplement, d'une part, des parties entre elles et, d'autre part, par rapport à la tête de vis pédiculaire correspondante (2). Ces agencements d'accouplement (1a1) et (1b1) sont constitués par deux fourchettes aptes à être reliées avec capacité d'articulation. Par exemple, les branches de l'une des fourchettes (1a1) présente intérieurement des ergots faisant office d'axe coopérant avec des échancrures que présente la face externe des branches de l'autre fourchette (1b1).

L'accouplement des deux parties (1a) et (1b), qui constituent donc des éléments demi-tubulaires, est réalisé de façon circulaire au contact de la face externe des têtes de vis pédiculaires (2).

Ces dispositions permettent d'écarter les parties (1a) et (1b) pour leur mise en place par rapport à la tête de vis pédiculaire (2), puis de les rabattre pour enserrer ladite tête de vis pédiculaire. A noter que la tête de vis pédiculaire (2) présente des agencements de retenue coopérant avec des agencements complémentaires d'au moins l'une des parties (1a) et (1b). Par exemple, ces agencements sont constitués par au moins un pion ou autre moyen d' indexation que présente l'une des parties (1a) et (1b) à proximité de la fourchette (1a1) ou (b1) et apte à coopérer avec un trou borgne que présente une partie de la tête de vis pédiculaire (2).

L'autre extrémité de chaque partie (1a) et (1b) coopère, après avoir été rabattue comme indiqué précédemment, avec un bouchon creux (6) apte à assurer la liaison desdites parties à constituer l'élément tubulaire en tant que tel. Après accouplement des parties (1a) et (1b), comme indiqué, ces dernières délimitent des lumières diamétralement opposées (1c) et (1d).

Les tiges d'alignement (4) et de manoeuvre (5) sont engagées dans les lumières (le) et (1d) avec capacité de déplacement parallèle à l'axe des éléments tubulaires (1).

Comme il sera indiqué dans la suite de la description, la tige de manoeuvre (5) est apte à agir, au fur et à mesure de son déplacement, sur un moyen (7) pour le déplacement concomitant de la tige cintrée implantable (3) préalablement engagée dans les lumières (le) et (1d).

Le moyen (7), pour le déplacement de la tige cintrée implantable (3), est constitué par une bague montée avec capacité de coulissement le long de l'élément tubulaire (1). Cette bague (7) présente à sa base des échancrures diamétralement opposées (7a) aptes à être positionnées en regard des lumières (1c) et (1d) pour coopérer avec la section de la tige cintrée (3). Ces dispositions assurent l'indexation de la tige cintrée (3) lui évitant de tourner sur elle-même, au fur et à mesure de son déplacement.

A noter également que les échancrures (7a) de la bague (7) coopèrent, après déplacement de ladite bague, avec un ergot que peut présenter une partie de l'élément tubulaire (1) pour assurer le blocage en position de ladite bague. Les tiges (4) et (5) sont disposées au-dessus des bagues (7) sous les bouchons (6), tandis que la tige cintrée implantable (3) est disposée sous lesdites bagues (7).

Il résulte donc de ces dispositions qu'en exerçant un appui sur la tige (5), on provoque le déplacement des différentes bagues (7) et, par conséquent, celui de la tige cintrée implantable (3) au travers des lumières (1c) et (1d) des éléments (1) jusqu'à ce que la tige cintrée (3) soit positionnée dans les têtes de vis pédiculaires correspondantes (2). Ce positionnement est possible étant donné que les têtes de vis (2) sont totalement dégagées suite à l'accouplement et l'alignement des éléments tubulaires (1) qui s'effectuent par l'extérieur desdites têtes de vis pédiculaires.

Selon une autre caractéristique importante de l'invention, la tige de manoeuvre (5) est destinée à être retirée puis accouplée à l'extérieur des éléments tubulaires (1) disposés en alignement. Dans ce but, l'extrémité de chaque élément tubulaire (1), à l'opposé de leur accouplement avec les têtes de vis pédiculaires (2), reçoit un collier rapporté (8) présentant une partie (8a) débordant latéralement pour l'engagement de la tige (5).

A noter que les différents éléments tubulaires (1) sont reliés, deux à deux, au niveau de leur extrémité libre, par un organe espaceur (9).

La longueur de cet organe espaceur (9) est variable, au choix du chirurgien ; cette longueur détermine la distance entre les éléments tubulaires qui conduit à déplacer les vertèbres entre elles ; ceci permet au chirurgien de contrôler la courbure de la section de colonne implantée, et par là-même de restaurer une courbure physiologique (par exemple cyphose thoracique).

Concernant les organes espaceurs, il est proposé une gamme de longueurs différentes.

Chaque organe espaceur permet de solidariser deux éléments tubulaires contigus dont la position forme un angle entre eux. Chaque organe espaceur est constitué d'une plaque présentant deux trous ronds munis chacun d'un anneau formant une rotule mobile, inséré dans l'épaisseur de la plaque. Le maintien intervient lorsqu'on glisse l'anneau le long de chaque élément tubulaire.

D'une manière connue, chaque tête de vis pédiculaire (2) est une douille avec une échancrure (2a) pour l'engagement de la tige cintrée implantable (3). La tête de vis pédiculaire (2) est prolongée, de part et d'autre de l'échancrure (2a), par des pattes sécables (2b) et (2c). De la même façon que l'alésage de la douille, les pattes (2b) et (2c) sont taraudées pour la mise en place d'un écrou (10) en vue de la fixation de la tige (3) dans la tête de vis pédiculaire correspondante.

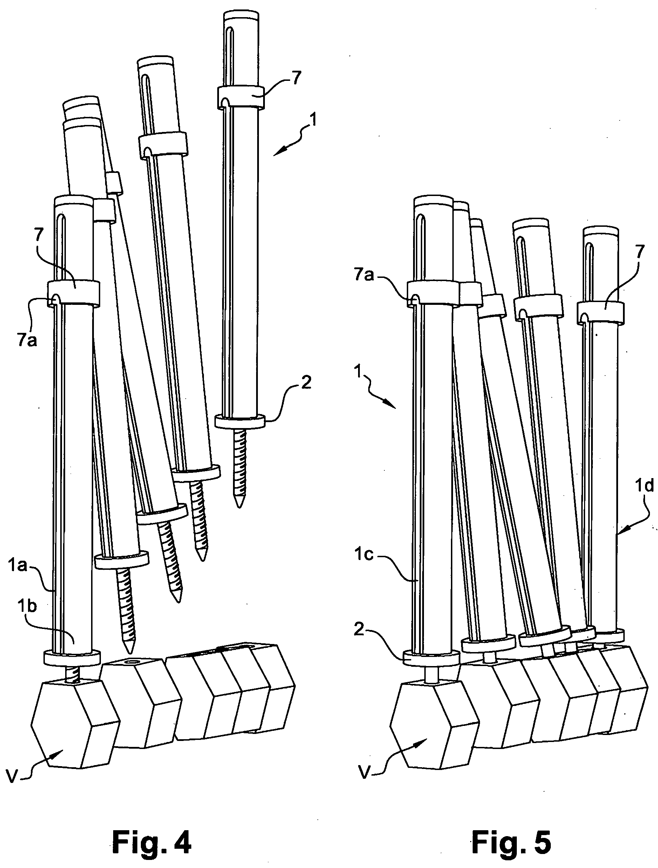

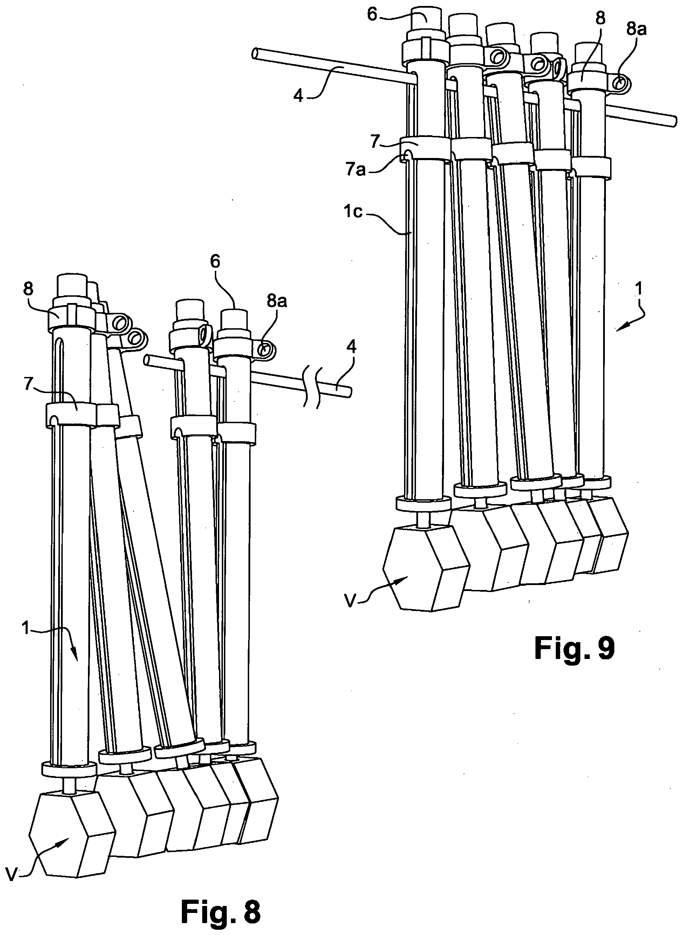

Compte tenu des caractéristiques à la base du dispositif de correction la méthode de correction est décrite ci-après en référence aux figures 4 à 20.

On accouple, sur chacune des têtes de vis (2), les éléments tubulaires (1), dans les conditions indiquées précédemment, c'est-à-dire sur l'extérieur des têtes de vis, pour dégager l'intérieur desdites têtes de vis.

Les différentes vis pédiculaires à ailettes longues (2), équipées des éléments tubulaires (1), sont fixées dans les corps vertébraux, par exemple du côté convexe. A noter que les têtes de vis pédiculaires peuvent être du type monobloc ou du type polyaxial, comme cela est parfaitement connu pour un homme du métier.

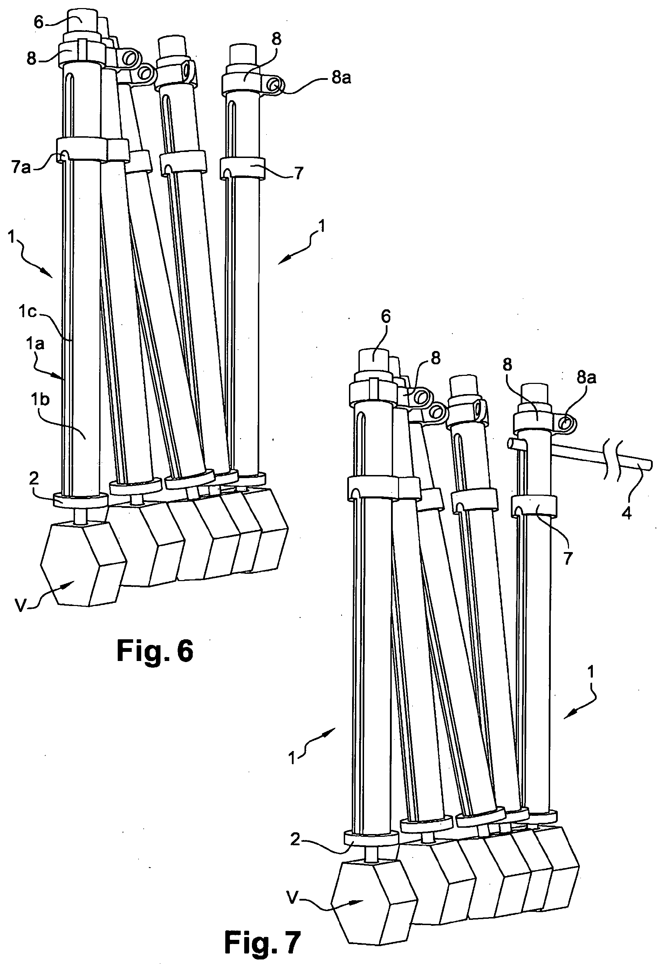

Chaque élément tubulaire (1) est ensuite équipé de son bouchon (écrou de fixation) (6). La bague (7) est positionnée en partie haute de l'élément tubulaire considéré. A ce stade, les éléments tubulaires (1) sont décalés angulairement en correspondance avec la déformation de la colonne vertébrale (figures 4 et 5). Les colliers (8) sont également disposés en partie haute des éléments tubulaires (figure 6).

On engage ensuite la tige (4) au travers des lumières (1c) et (1d) des différents éléments tubulaires (1) pour provoquer leur redressement progressif en vue de leur alignement en générant une translation, une bascule et une rotation des vertèbres pour la correction de la colonne (figures 7, 8 et 9).

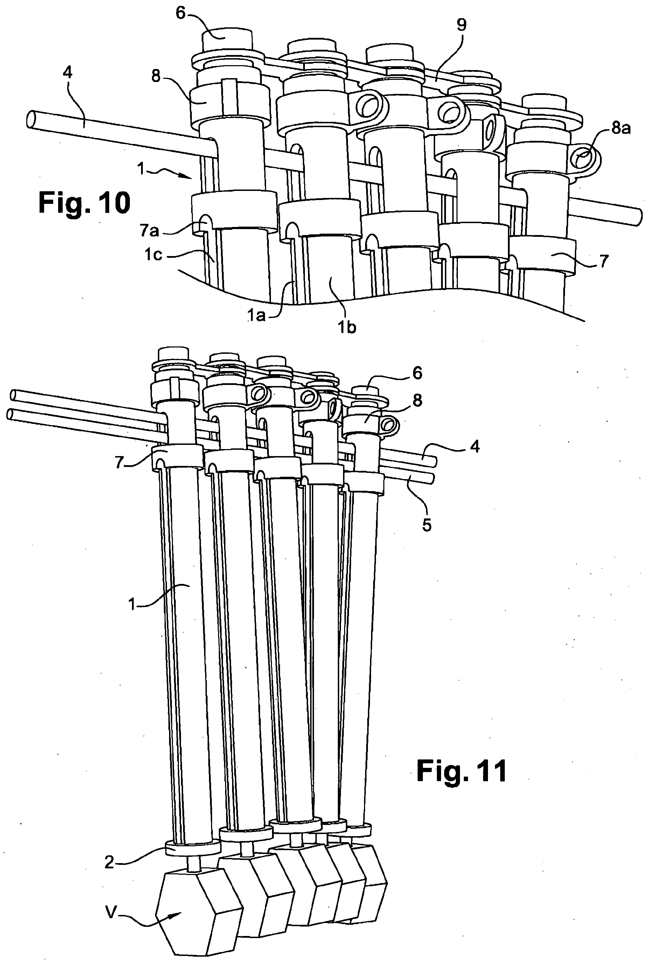

Les différents éléments tubulaires (1) sont ensuite accouplés, deux à deux, par les organes espaceurs (9) (figure 10), afin de prédéterminer la correction de courbure attendue, telle qu'une cyphose thoracique par exemple.

On engage transversalement, sous la tige d'alignement (4), la tige de manoeuvre (5) apte à prendre appui sur les bagues (7) (figure 11).

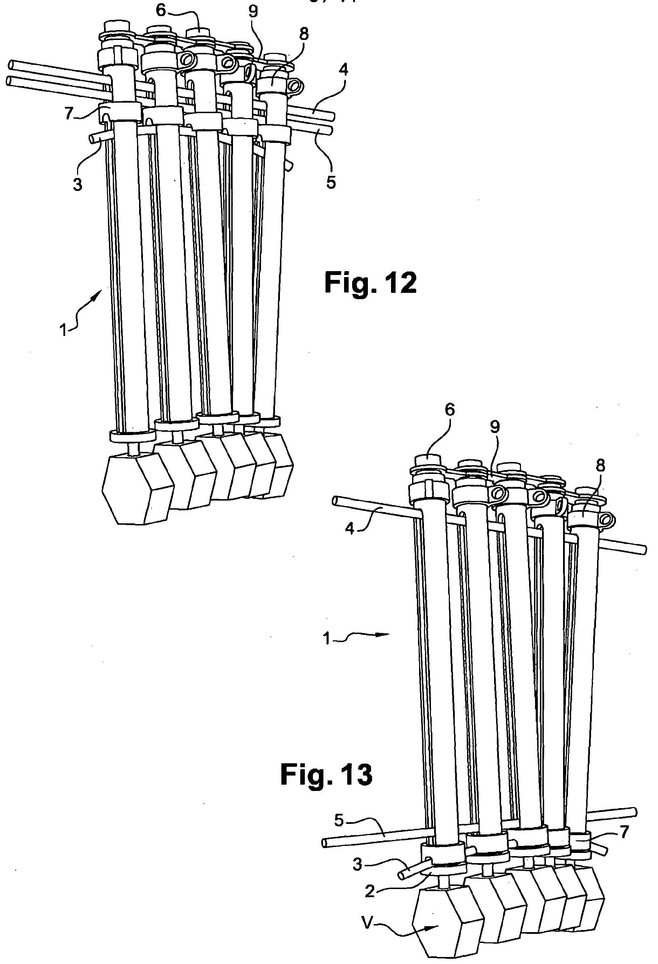

On engage transversalement, sous les bagues (5) (figure 12), la tige cintrée implantable (3) en vue de son positionnement et sa fixation dans les têtes de vis pédiculaires (2a). Dans ce but, on agit en poussée, selon un mouvement dirigé de haut en bas, sur la tige de manoeuvre (5) pour provoquer le déplacement des différentes bagues (7) et le déplacement concomitant de la tige cintrée (3) jusqu'à ce que cette dernière vienne se positionner dans les échancrures des douilles des têtes de vis pédiculaires (figure 13).

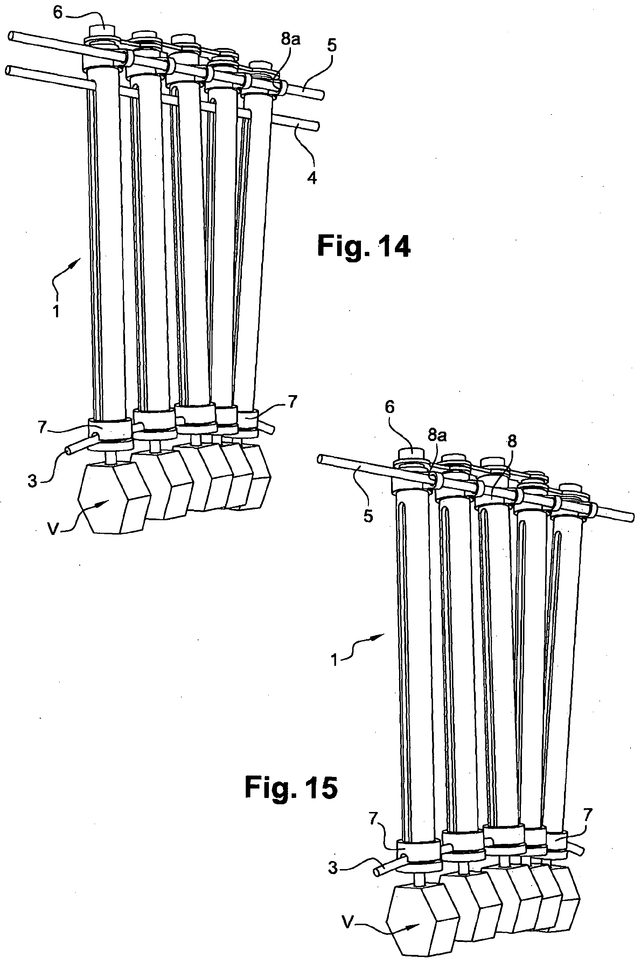

On retire ensuite la tige de manoeuvre (5) pour la positionner dans les différents colliers (8), c'est-à-dire à l'extérieur des éléments tubulaires (1) (figure 14). Il est alors possible de retirer la tige d'alignement (4) étant donné que la présente correction de courbure de la colonne est maintenue par la tige de manoeuvre (5) disposée à l'extérieur des éléments tubulaires, comme indiqué (figure 15).

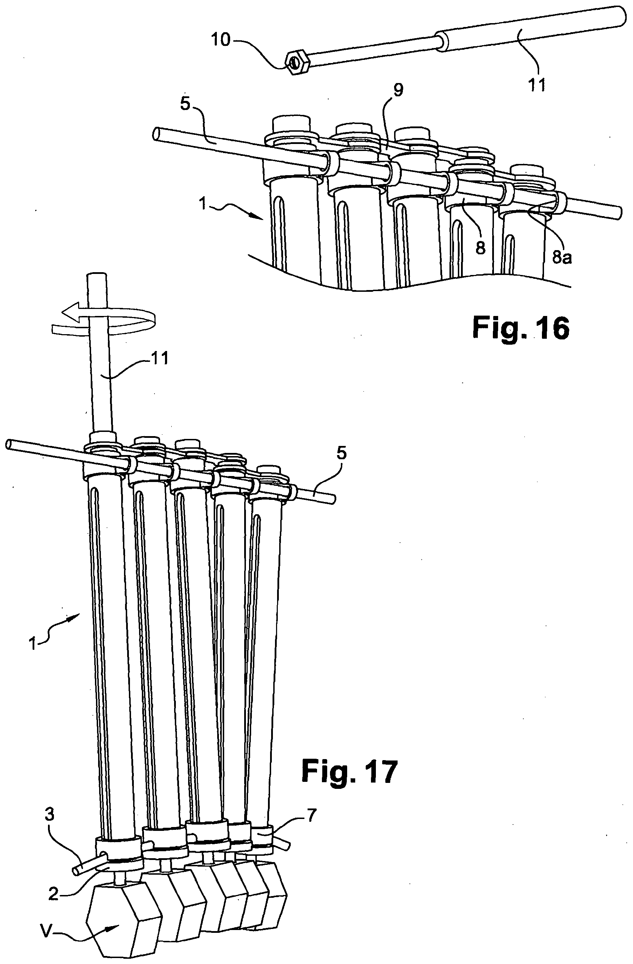

Il résulte de ces dispositions que l'intérieur des différents éléments tubulaires (1) et des têtes de vis pédiculaires (2), est totalement libre permettant, par conséquent, l'introduction des écrous (10) au travers des différents éléments tubulaires (1) par un organe de manoeuvre approprié (11) (figure 16) et de visser les écrous (10) dans les têtes de vis pédiculaires pour assurer la fixation de la tige implantable (3) (figure 17).

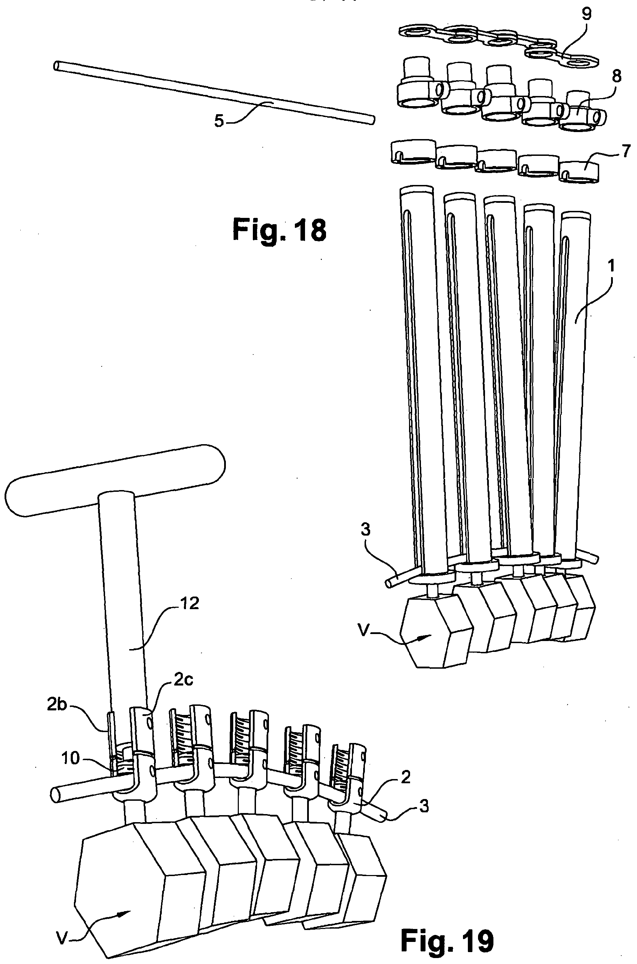

A ce stade du mode opératoire, il est alors possible de démonter le dispositif et, par conséquent, de retirer les éléments tubulaires (figure 18).

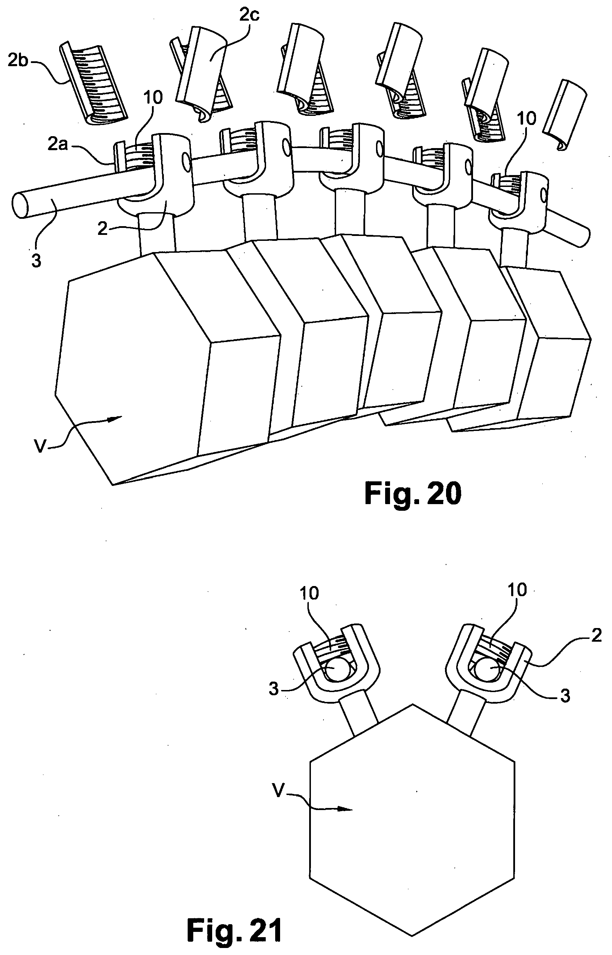

Il suffit ensuite d'assurer le vissage complet des écrous (10) au moyen d'un organe (12) pour assurer la liaison et la fixation définitive de la tige cintrée (3) dans les têtes de vis pédiculaires (2) (figure 19), de casser les parties sécables (2b) et (2c) des vis pédiculaires (2) (figure 20).

Il est ensuite possible de procéder de la même façon à partir du côté convexe (figure 21).

Les avantages ressortent bien de la description, en particulier on souligne et on rappelle que chaque côté de la colonne est corrigé en une seule fois, en procédant au redressement des vertèbres, puis à la mise en place de la tige cintrée implantable et à sa fixation, sans être obligé de démonter le dispositif de correction permettant, non seulement ,de gagner du temps, mais, surtout, d'éviter de perdre de la correction.

A noter également que les organes espaceurs, entre les différents tubes, sont très importants pour restaurer une cyphose, permettant au chirurgien de régler l'écartement des tubes et, par conséquent, d'agir sur ladite cyphose.

Sans pour cela sortir du cadre de l'invention, on peut prévoir une utilisation symétrique, à savoir deux côtés instrumentés pour une meilleure efficacité de la force de correction. On peut également imaginer des moyens de liaison entre les cotés en liant horizontalement les éléments tubulaires ou tiges de chaque côté.

De même, on peut envisager, l'utilisation avec une voie d'abord mini invasive/percutanée : les incisions pratiquées sur la peau sont limitées au diamètre de chaque élément tubulaire, la tige de manoeuvre est amenée du haut vers le bas jusqu'au contact de la peau : la position des vertèbres est corrigée « sous la peau».

On peut réaliser une incision à distance, en haut ou en bas de la ligne formée par l'ensemble des éléments tubulaires et on glisse la tige implant cintrée sous la peau jusqu'à atteindre l'intérieur desdits éléments et des têtes de vis (position finale de la tige implant).

## Revendications

1. Dispositif chirurgical pour la correction de la déformation de la colonne vertébrale comprenant des éléments tubulaires (1) aptes à être fixés temporairement au niveau de têtes de vis pédiculaires agencées pour être reliées par une tige cintrée implantable (3), et des tiges (4) et (5) qui sont engagées au travers des différents éléments tubulaires (1) pour les aligner en vue de la correction de la dite colonne, par translation, bascule et rotation des vertèbres,
**caractérisé en ce que** le dispositif comprend un moyen (7) pour le déplacement concomitant de la tige cintrée (3), et des colliers rapportés (8) à l'extérieur des éléments tubulaires, et **en ce que** chaque élément tubulaire (1) est en deux parties indépendantes (1a) et (1b), pour être accouplé à l'extérieur des têtes de vis pédiculaires (2), l'une des extrémités de chaque partie présentant des agencements d'accouplement (1a1) et (1b1) desdites parties entre elles et par rapport à la tête de vis pédiculaire correspondante (2), l'une des tiges (4) étant pour l'alignement des éléments tubulairs, et l'autre (5), étant pour le déplacement de la tige cintrée implantable, étant engagées dans des lumières (le) et (1d) délimitées par les deux parties de l'éléments tubulaires avec capacité de déplacement parallèle à l'axe des éléments tubulaires (1), la tige de déplacement (5) étant apte à agir, au fur et à mesure de son déplacement, sur le moyen (7) pour le déplacement concomitant de la tige cintrée (3), préalablement engagée dans les lumières (le) et (1d), en direction des têtes de vis pédiculaires (2), ladite tige de déplacement (5) étant destinée à être retirée puis accouplée au moyen des colliers rapportés (8) à l'extérieur des éléments tubulaires (1) disposés en alignement pour permettre le retrait de la tige d'alignement (4) et l'engagement, à l'intérieur de chaque élément tubulaire, d'un organe de manoeuvre (11) d'un écrou (10) de fixation de la tige cintrée dans chacune des têtes de vis pédiculaires (2).

2. Dispositif selon la revendication 1, **caractérisé en ce que** les agencements d'accouplement (1a1) et (1b1) sont constitués par deux fourchettes aptes à être reliées avec capacité d'articulation pour permettre d'écarter les parties pour leur mise en place par rapport à la tête de vis pédiculaire puis les rabattre pour enserrer ladite tête maintenue entre lesdites parties par des moyens de retenue.

3. Dispositif selon l'une quelconque des revendications 1 et 2, **caractérisé en ce qu'**il comprend des écrous creux de liaison et **en ce que** l'extrémité de chaque partie (1a) et (1b) considérée à l'opposé des agencements d'accouplement (1a1) et (1a2) coopère, après avoir été rabattue, avec un des écrous creux de liaison (6) pour constituer l'élément tubulaire en tant que tel, lesdites parties (1a) et (1b) délimitant les lumières diamétralement opposées (1c) et (1d).

4. Dispositif selon la revendication 1, **caractérisé en ce que** le moyen (7) de déplacement de la tige cintrée implantable (3), est une bague montée avec capacité de coulissement le long de l'élément tubulaire (1).

5. Dispositif selon la revendication 4, **caractérisé en ce que** la bague (7) présente, à sa base, des échancrures diamétralement opposées (7a) aptes à être positionnées en regard des lumières (1c) et (1d) pour coopérer avec la section de la tige cintrée (3).

6. Dispositif selon l'une quelconque des revendications 4 et 5, **caractérisé en ce que** les échancrures (7a) de la bague (7) coopèrent, avant déplacement de ladite bague, avec un ergot de blocage en position, les tiges d'alignement (4) et de déplacement (5) étant disposées au-dessus de la bague (7), tandis que la tige cintrée implantable (3) est disposée sous ladite bague (7).

7. Dispositif selon la revendication 1, **caractérisé en ce que** l'extrémité de chaque élément tubulaire (1), à l'opposé de son accouplement avec la tête de vis pédiculaire (2), reçoit le collier rapporté (8) présentent une partie (8a) débordant latéralement pour l'engagement de la tige de déplacement (5) ayant servi à déplacer la bague (7) et, d'une manière concomitante, la tige cintrée implantable (3).

8. Dispositif selon la revendication 1, **caractérisé en ce que** chaque tête de vis pédiculaire (2) présente, d'une manière connue, une échancrure (2a) en constituant une douille pour l'engagement de la tige cintrée (3), ladite tête étant prolongée, de part et d'autre de l'échancrure, par des pattes sécables et taraudées (2b) et (2c) pour la mise en place d'un écrou (10) de fixation de la tige cintrée dans la tête de vis pédiculaire correspondante.

9. Dispositif selon la revendication 8, **caractérisé en ce que** la tête de vis pédiculaire (2) est du type monobloc ou polyaxial.

10. Dispositif selon la revendication 1, **caractérisé en ce qu'**il comprend des organes espaceurs (9) qui sont disposés au niveau de l'extrémité libre des éléments tubulaires pour les relier deux à deux.

11. Dispositif selon la revendication 10, **caractérisé en ce que** chaque organe espaceur est constitué d'une plaque présentant deux trous ronds munis chacun d'un anneau formant une rotule mobile, inséré dans l'épaisseur de la plaque.

## Patentansprüche

1. Chirurgische Vorrichtung zur Korrektur der Wirbelsäulenverkrümmung mit Hohlkörperelementen (1), die vorübergehend an Köpfen von Pedikelschrauben befestigt werden können, welche so ausgelegt sind, dass sie über einen implantierbaren, gebogenen Stab (3) miteinander verbunden werden können, und mit Stäben (4) und (5), die durch die verschiedenen Hohlkörperelemente (1) gesteckt werden, um diese zur Korrektur der Wirbelsäule durch Translation, Kippen oder Rotation der Wirbel zueinander auszurichten,
**dadurch gekennzeichnet, dass** die Vorrichtung ein Mittel (7) zur gleichzeitigen Bewegung des gebogenen Stabs (3), und außerhalb der Hohlkörperelemente aufgebrachte Schellen (8) umfasst, und dass jedes Hohlkörperelement (1) zur Verbindung außerhalb der Pedikelschraubenköpfe (2) aus zwei unabhängigen Teilen (1a) und (1b) besteht, wobei eines der Enden jeden Teils Verbindungsanordnungen (1a1) und (1b1) zur Verbindung der Teile untereinander und bezogen auf den jeweiligen Pedikelschraubenkopf (2) umfasst, wobei einer der Stäbe (4) der Ausrichtung der Hohlkörperelemente und der andere (5) der Bewegung des implantierbaren, gebogenen Stabs dient, welche durch die Schlitze (1c) und (1d) gesteckt werden, die durch die beiden Teile der Hohlkörperelemente begrenzt werden und parallel zur Achsrichtung der Hohlkörperelemente (1) bewegbar sind, wobei der Bewegungsstab (5) während seiner Fortbewegung in der Lage ist, zur gleichzeitigen Bewegung des gebogenen Stabs (3), der zuvor durch die Schlitze (1c) und (1d) gesteckt wurde, hin zu den Pedikelschraubenköpfen (2) auf ein Mittel (7) einzuwirken, wobei der Bewegungsstab (5) dazu bestimmt ist, herausgezogen und dann über die aufgebrachten Schellen (8) außerhalb der fluchtend ausgerichteten Hohlkörperelemente (1) gesteckt zu werden, um ein Herausziehen des Ausrichtungsstabs (4) und, in jedem Hohlkörperelement, das Einstecken eines Elementes (11) zur Betätigung einer Mutter (10) zur Befestigung des gebogenen Stabs in jedem der Pedikelschraubenköpfe (2) zu ermöglichen.

2. Vorrichtung nach Anspruch 1, **dadurch gekennzeichnet, dass** die Verbindungsanordnungen (1a1) und (1b1) von zwei verbindbaren, gelenkigen Gabeln gebildet werden, so dass die Teile beim Aufsetzen auf den Kopf der Pedikelschraube auseinandergespreizt und dann wieder zusammengeführt werden können, um den zwischen den Teilen eingefassten Kopf durch Rückhaltemittel einzuspannen.

3. Vorrichtung nach einem der Ansprüche 1 und 2, **dadurch gekennzeichnet, dass** die Vorrichtung hohle Überwurfmuttern enthält und dass das Ende des jeweils betrachteten Teils (1a) und (1b) auf der den Verbindungsanordnungen (1a1) und (1a2) gegenüberliegenden Seite mit einer der hohlen Überwurfmuttern (6) zusammenwirkt, um das Hohlkörperelement als solches zu bilden, wobei die Teile (1a) et (1b) die einander diametral gegenüberliegenden Schlitze (1c) und (1d) begrenzen.

4. Vorrichtung nach Anspruch 1, **dadurch gekennzeichnet, dass** es sich bei dem Mittel (7) zur Bewegung des implantierbaren, gebogenen Stabs (3) um eine Buchse handelt, die entlang des Hohlkörperelements (1) verschiebbar montiert ist.

5. Vorrichtung nach Anspruch 4, **dadurch gekennzeichnet, dass** die Buchse (7) an ihrer Basis einander diametral gegenüberliegende Aussparungen (7a) aufweist, die auf der Höhe der Schlitze (1c) und (1d) positioniert werden können, um mit dem Querschnitt des gebogenen Stabs (3) zusammenzuwirken.

6. Vorrichtung nach einem der Ansprüche 4 und 5, **dadurch gekennzeichnet, dass** die Aussparungen (7a) der Buchse (7) vor Bewegung der Buchse mit einem in Position befindlichen Arretierstift zusammenwirken, wobei der Ausrichtungsstab (4) und der Bewegungsstab (5) oberhalb der Buchse (7), der implantierbare, gebogene Stab (3) jedoch unterhalb der Buchse (7) angeordnet sind.

7. Vorrichtung nach Anspruch 1, **dadurch gekennzeichnet, dass** das Ende jeden Hohlkörperelements (1) auf der der Verbindung mit dem Pedikelschraubenkopf (2) gegenüberliegenden Seite die aufgebrachte Schelle (8) aufnimmt, die zum Einstecken des Bewegungsstabs (5), der zur Bewegung der Buchse (7) und gleichzeitig des implantierbaren, gebogenen Stabs (3) diente, einen seitlich überstehenden Teil (8a) aufweist.

8. Vorrichtung nach Anspruch 1, **dadurch gekennzeichnet, dass** jeder Pedikelschraubenkopf (2) in an sich bekannter Weise zum Einstecken des gebogenen Stabs (3) eine eine Hülse bildende Aussparung (2a) aufweist, wobei der Kopf zum Einsetzen einer Mutter (10) zur Befestigung des gebogenen Stabs in den entsprechenden Pedikelschraubenkopf zu beiden Seiten der Aussparung durch trennbare und mit Gewinden versehene Klauen (2b) und (2c) verlängert wird.

9. Vorrichtung nach Anspruch 8, **dadurch gekennzeichnet, dass** der Pedikelschraubenkopf (2) vom Typ her aus einem Stück besteht oder polyaxial ausgebildet ist.

10. Vorrichtung nach Anspruch 1, **dadurch gekennzeichnet, dass** die Vorrichtung Abstandshalter (9) aufweist, die am freien Ende der Hohlkörperelemente zu deren paarweisen Verbindung angeordnet sind.

11. Vorrichtung nach Anspruch 10, **dadurch gekennzeichnet, dass** jeder Abstandshalter aus einer Platte mit zwei runden Löchern gebildet wird, wobei jedes Loch einen ein bewegliches Kugelgelenk bildenden Ring aufweist, der in die Platte eingelassen ist.

## Claims

1. A surgical device for correcting the deformation of the spine, comprising tubular elements (1) adapted to be temporarily fixed at pedicle screw heads arranged to be connected by an implantable curved rod (3), and rods (4) and (5) which are engaged through the various tubular elements (1) to align them for correcting said column, by translation, tilt and rotation of the vertebrae,
**characterized in that** the device comprises means (7) for the concomitant displacement of the curved rod (3), and attached collars (8) outside the tubular elements, and **in that** each tubular element (1) is into two independent parts (1a) and (1b) to be coupled to the outside of the pedicle screw heads (2), one end of each portion having arrangements (1a1) and (1b1) for coupling said parts with each other and relative to the corresponding pedicle screw head (2), one of the rods (4) being for aligning the tubular elements, and the other (5) being for displacing the implantable curved rod, inserted into slots (1c) and (1d) defined by the two parts of the tubular elements such that they can move parallel to the axis of the tubular elements (1), the displacement rod (5) being being suitable, as it moves, to act on means (7) for the concomitant displacement of the curved rod (3), previously inserted in the slots (1c) and (1d), toward the pedicle screw heads (2 ), said displacement rod (5) being intended to be removed and coupled through reported collars (8) outside of the tubular elements (1) arranged in alignment in order to enable the withdrawal of the alignment rod (4) and the insertion inside each tubular element of an actuating member (11) of a nut (10) for fixing the curved rod in each of the pedicle screw heads (2).

2. The device according to claim 1, **characterized in that** the arrangements (1a1) and (1b1) for coupling are formed by two forks suitable to be connected with articulation capability to allow to spread the parts for their positionning relative to the pedicle screw head, and then to swivel them to clamp said head held between said parts by retaining means.

3. The device according to claim 1 or 2, **characterized in that** it comprises connecting hollow nuts and **in that** the end of each part (1a) and (1b) considered as opposed to arrangements (1a1) and (1a2) for coupling cooperates, after being folded down, with a connecting hollow nuts (6) to form the tubular element as such, said parts (1a) and (1b) defining diametrically opposed slots(1c) and (1d).

4. The device according to claim 1, **characterized in that** the means (7) for moving the implantable curved rod (3) is a ring mounted slidably along the tubular element (1).

5. The device according to claim 4, **characterized in that** the ring (7) has, at its base, diametrically opposed notches (7a) suitables to be positioned opposite the slots (1c) and (1d) to cooperate with the section of the curved rod (3).

6. The device according to any one of claims 4 and 5, **characterized in that** the notches (7a) of the ring (7) cooperate, before movement of said ring, with a locking lug in position, the alignment (4) and displacement (5) rods being disposed above the ring (7), while the implantable curved rod (3) is disposed below said ring (7).

7. The device according to claim 1, **characterized in that** the end of each tubular element (1), at the opposite to its coupling with the pedicle screw head (2), receives the attached collar (8) having a part (8a) projecting laterally for the insertion of the displacement rod (5) used to move the ring (7), and concomitantly the implantable curved rod (3).

8. The device according to claim 1, **characterized in that** each pedicle screw head (2) has in a known manner a notch (2a) for constituting a socket for the insertion of the curved rod (3), said head being extended on both sides of the notch, by tapped and breakable tabs (2b) and (2c) for the positionning of a nut (10) for fixing the curved rod in the corresponding pedicle screw head.

9. The device according to claim 8, **characterized in that** the pedicle screw head (2) is of one-piece or polyaxial type.

10. The device according to claim 1, **characterized in that** it comprises spacer members (9) which are disposed at the free end of the tubular elements to connect them in pairs.

11. The device according to claim 10, **characterized in that** each spacer member is constituted in a plate having two round holes each provided with a ring forming a movable ball, inserted in the thickness of the plate.
